# EUROPEAN PATENT SPECIFICATION

(11) **EP 2 090 653 B1**
(45) Date of publication and mention of the grant of the patent: **31.08.2011**
(21) Application number: 07832502.4
(22) Date of filing: 26.11.2007
(51) Int. Cl.: C12N 15/00, C12N 15/09, C12Q 1/68, C12Q 1/70

(54) **METHOD FOR DETECTION OF APPLE MOSAIC VIRUS, PRIMER SET FOR THE DETECTION, AND KIT FOR THE DETECTION**
VERFAHREN ZUM NACHWEIS VON APFELMOSAIKVIRUS, PRIMERSATZ FÜR DEN NACHWEIS UND KIT FÜR DEN NACHWEIS
PROCEDE DE DETECTION DU VIRUS DE LA MOSAIQUE, ENSEMBLE D'AMORCES ET NECESSAIRE DESTINES A CETTE DETECTION

(30) Priority: 30.11.2006 JP 2006324371
(43) Date of publication of application: 19.08.2009
(73) Proprietor: Sapporo Breweries Limited, Tokyo 150-8522 (JP)
(72) Inventor: OKADA, Yukio, Tokyo 150-8522 (JP); ITOGA, Yutaka, Tokyo 150-8522 (JP)
(74) Representative: Vossius & Partner
(86) International application number: PCT/JP2007/072777
(87) International publication number: WO 2008/066002

(56) References cited:
- EP-A1- 1 020 534
- JP-A- 2004 089 180
- SÁNCHEZ-NAVARRO J A ET AL: "Simultaneous detection and identification of eight stone fruit viruses by one-step RT-PCR" EUROPEAN JOURNAL OF PLANT PATHOLOGY, KLUWER ACADEMIC PUBLISHERS, DO LNKD- DOI:10.1007/S10658-004-1422-Y, vol. 111, no. 1, 1 January 2005 (2005-01-01), pages 77-84, XP019239227 ISSN: 1573-8469
- HASSAN M ET AL: "Simultaneous detection and identification of four pome fruit viruses by one-tube pentaplex RT-PCR" JOURNAL OF VIROLOGICAL METHODS, ELSEVIER BV, NL LNKD- DOI:10.1016/J.JVIROMET.2005.11.002, vol. 133, no. 2, 1 May 2006 (2006-05-01), pages 124-129, XP025030327 ISSN: 0166-0934 [retrieved on 2006-05-01]
- AOI Y ET AL: "Real-time quantitative LAMP (loop-mediated isothermal amplification of DNA) as a simple method for monitoring ammonia-oxidizing bacteria" JOURNAL OF BIOTECHNOLOGY, ELSEVIER SCIENCE PUBLISHERS, AMSTERDAM, NL LNKD- DOI:10.1016/J.JBIOTEC.2006.04.007, vol. 125, no. 4, 1 October 2006 (2006-10-01), pages 484-491, XP024956642 ISSN: 0168-1656 [retrieved on 2006-10-01]
- PETRZIK K. ET AL.: 'Screening of apple mosaic virus in hop cultivars in the Czech Republic by reverse transcription-polymerase chain reaction' ACTA VIROLOGICA vol. 41, no. 2, 1997, pages 101 - 103, XP003022686

## Description

### Technical Field

The present invention relates to a detection method, a detection primer set and a detection kit for apple mosaic virus.

### Background Art

Apple mosaic virus is a phytopathogenic virus belonging to the genus *Bromovirus.,* and it widely infects hops (*Humulus lupulus L*.) and apples (*Malus domestica Malus*). Once hops and apples have been infected with apple mosaic virus, the infectious damage spreads throughout the hops primarily by mechanical contact.

In major hop-producing countries such as Germany, the United States, England and the Czech Republic, and in Japan, virus-free shoots produced by shoot tip culture are used for hop cultivation to prevent infectious damage by apple mosaic virus.

In hop cultivation farms, apple mosaic virus infection is periodically examined to halt secondary infection with apple mosaic virus. The examination of apple mosaic virus infection generally involves detection of hop-infecting apple mosaic virus by ELISA. Petrzik K. et al; (Acta Virologica 41:101-103 (1997)) describes a method for screening of apple mosaic virus in hop cultivars in the Czech Republic by using two specific primers.

The detection method for apple mosaic virus by ELISA comprises a step of reacting apple mosaic virus particles in a specimen with a primary antibody that specifically recognizes apple mosaic virus on a microplate containing the primary antibody in solid phase, a step of washing off the components in the specimen that have not bound to the primary antibody, a step of reacting the apple mosaic virus particles that have bound to the primary antibody with a secondary antibody that is enzyme-labeled and specifically recognizes apple mosaic virus, a step of washing off the secondary antibody that has not bound to the apple mosaic virus particles, a step of performing enzyme reaction between the enzyme labeling the secondary antibody and a chemichromic or chemiluminescent substrate, and a step of estimating the amount of apple mosaic virus particles based on the intensity of coloring or luminescence obtained by the enzyme reaction.

[Non-patent document 1] Pethybridge et al., 2004, Australian Journal of Agricultural Research, Vol. 55, p.765-770

### Disclosure of the Invention

### Problems to be Solved by the Invention

When the titer or specificity of the apple mosaic virus-recognizing antibody used in ELISA is inadequate, it is affected by components in the specimen other than apple mosaic virus, and this may cause signal indicating the presence of apple mosaic virus to be evaluated as background signal, or nonspecific binding to be evaluated as signal indicating the presence of apple mosaic virus.

In addition, since detection methods for apple mosaic virus by ELISA comprise multiple steps as explained above, each of the steps must be carried out in the laboratory under the same conditions including reaction time and washing frequency, and if the number of specimens is increased the operation of each step becomes more complex and precise evaluation tends to be hindered.

Furthermore, because ELISA methods perform detection using viral particles themselves as the target, they cannot detect proviruses that are incorporated in viral genomes or host genomes, while hops infected with apple mosaic virus are often judged to be false-positive or undetectable in summer season in which higher temperature is known to notably reduce apple mosaic virus particle content.

In light of these problems, it is an object of the present invention to provide a method for detecting apple mosaic virus specifically and with high sensitivity in any season including during cultivation, harvesting and storage, regardless of the performance (titer and specificity) of the antibody used to detect the apple mosaic virus, the number of specimens and the location of evaluation. It is a further object of the invention to provide a detection primer set and detection kit for apple mosaic virus using the method.

### Means for Solving the Problems

In order to achieve the objects stated above, the invention provides a detection method for apple mosaic virus that comprises an extraction step in which apple mosaic virus RNA is extracted from a specimen, an amplification step in which a primer set containing 4 different oligonucleotides consisting of the nucleotide sequences represented by SEQ ID NO: 1-4 of the Sequence Listing is used for amplification of cDNA by Reverse Transcription-Loop-mediated isothermal AMPlification (RT-LAMP) using the RNA as template, and a judging step in which apple mosaic virus is judged to be present when amplification of cDNA containing the nucleotide sequence represented by SEQ ID NO: 8 of the Sequence Listing has occurred in the amplification step.

The present inventors have discovered that apple mosaic virus can be detected in a simple and highly sensitive manner by utilizing Reverse Transcription-Loop-mediated isothermal amplification (RT-LAMP) as an isothermal gene amplification reaction to amplify a specific region of the gene for the coat protein of apple mosaic virus. The term "RT-LAMP" refers to a method of amplifying cDNA complementary to an RNA template, incorporating reverse transcription reaction and isothermal gene amplification reaction by LAMP. The principle of this method is laid out in detail in International Patent Publication No. WO00/28082.

If RNA is extracted from tissue of hops for the purpose of determining the presence of apple mosaic virus infection, and a primer set containing 4 different oligonucleotides consisting of the nucleotide sequences represented by SEQ ID NO: 1-4 of the Sequence Listing is added to the extract for amplification of cDNA by RT-LAMP, according to the detection method described above, it is possible to detect cDNA of a partial sequence of genomic RNA of apple mosaic virus in a simple and highly sensitive manner, thus allowing the presence of apple mosaic virus infection to be determined.

The nucleotide sequence of genomic RNA of apple mosaic virus complementary to the nucleotide sequence represented by SEQ ID NO: 8 of the Sequence Listing, and the nucleotide sequences of genomic RNA of apple mosaic virus to which the 4 different oligonucleotides consisting of the nucleotide sequences represented by SEQ ID NO: 1-4 of the Sequence Listing anneal, are partial sequences of the gene for the coat protein of apple mosaic virus, and the regions of the genomic RNA of apple mosaic virus containing these nucleotide sequences are suitable target regions for amplification of cDNA by RT-LAMP. Consequently, the presence of apple mosaic virus can be specifically detected by confirming whether or not amplified cDNA is present.

The amplification step is preferably a step in which a primer set containing 6 different oligonucleotides consisting of the nucleotide sequences represented by SEQ ID NO: 1-6 of the Sequence Listing is used for amplification of cDNA by Reverse Transcription-Loop-mediated isothermal AMPlification (RT-LAMP) using the RNA as template.

If the cDNA is amplified with addition of 2 different oligonucleotides consisting of the nucleotide sequences represented by SEQ ID NO: 5 and 6 of the Sequence Listing to the 4 different oligonucleotides consisting of the nucleotide sequences represented by SEQ ID NO: 1-4 of the Sequence Listing, as Loop primers, the number of DNA synthesis origins will be increased, thus shortening the amplification time and allowing more efficient detection of apple mosaic virus.

The invention further provides a detection primer set for apple mosaic virus that comprises 4 different oligonucleotides consisting of the nucleotide sequences represented by SEQ ID NO: 1-4 of the Sequence Listing or 6 different oligonucleotides consisting of the nucleotide sequences represented by SEQ ID NO: 1-6 of the Sequence Listing.

If RNA is extracted from tissue of hops for the purpose of determining the presence of apple mosaic virus infection, and the aforementioned primer set is added to the extract for amplification of cDNA by RT-LAMP, it is possible to detect cDNA of a partial sequence of genomic RNA of apple mosaic virus in a simple and highly sensitive manner, thus allowing the presence of apple mosaic virus infection to be determined.

The invention still further provides a detection kit for apple mosaic virus which contains a detection primer set for apple mosaic virus comprising 4 different oligonucleotides consisting of the nucleotide sequences represented by SEQ ID NO: 1-4 of the Sequence Listing or 6 different oligonucleotides consisting of the nucleotide sequences represented by SEQ ID NO: 1-6 of the Sequence Listing, together with reverse transcriptase, strand displacing DNA synthase, dNTPs and buffer.

Using such a detection kit, it is possible to simultaneously procure the reagents necessary for detection of apple mosaic virus by RT-LAMP, and detection of apple mosaic virus can be easily accomplished without requiring complex procedures for preparation of reagent concentrations, even when the apple mosaic virus detection is in the field, for example.

### Effect of the Invention

According to the invention, it is possible to detect apple mosaic virus specifically and with high sensitivity in any season including during cultivation, harvesting and storage, regardless of the number of specimens or the location of evaluation. Moreover, the detection primer set and detection kit for apple mosaic virus according to the invention may be suitably used for RT-LAMP, thus allowing simple and high-sensitivity detection of apple mosaic virus.

### Brief Description of the Drawings

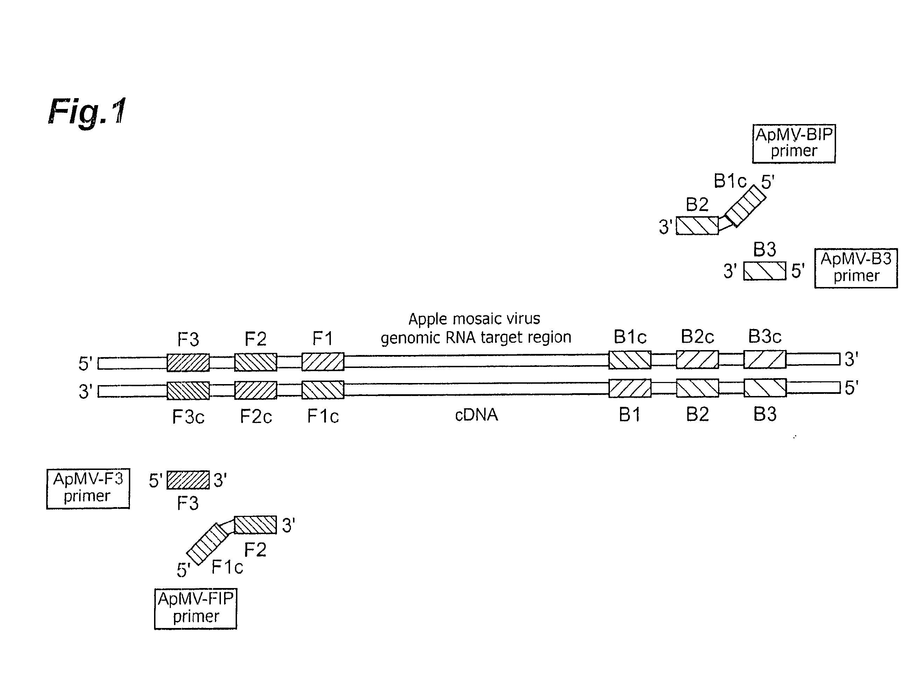

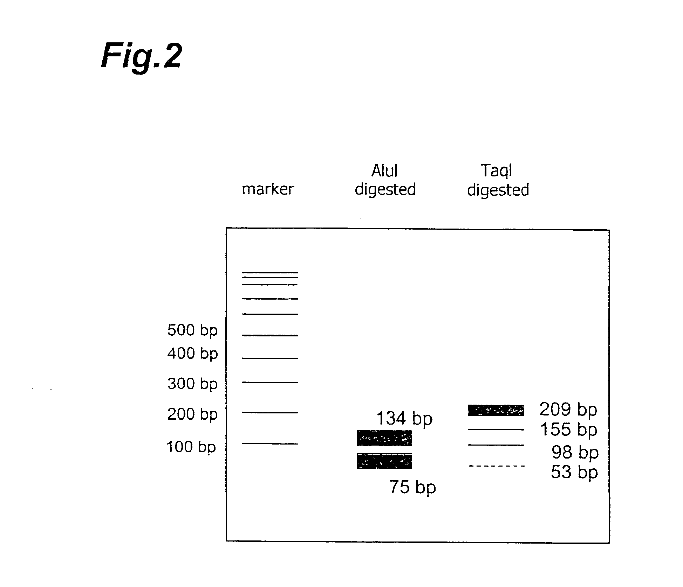

Fig. 1 is a diagram showing the positional relationship between the primer set comprising 4 different oligonucleotides consisting of the nucleotide sequences represented by SEQ ID NO: 1-4 of the Sequence Listing, and the target region of apple mosaic virus genomic RNA. Fig. 2 is a gel diagram showing the band pattern obtained by using restriction enzyme AluI or TaqI for digestion of cDNA amplified by RT-LAMP using an RNA-diluted sample of an apple mosaic virus detection specimen, followed by polyacrylamide gel electrophoresis.

### Best Mode for Carrying Out the Invention

Preferred embodiments of the invention will now be described in detail.

The detection method for apple mosaic virus according to the invention is characterized by comprising an extraction step in which apple mosaic virus RNA is extracted from a specimen, an amplification step in which a primer set containing 4 different oligonucleotides consisting of the nucleotide sequences represented by SEQ ID NO: 1-4 of the Sequence Listing is used for amplification of cDNA by Reverse Transcription-Loop-mediated isothermal AMPlification (RT-LAMP) using the RNA as template, and a judging step in which apple mosaic virus is judged to be present when amplification of cDNA containing the nucleotide sequence represented by SEQ ID NO: 8 of the Sequence Listing has occurred in the amplification step.

"Apple mosaic virus" is Apple mosaic ilarvirus which is an RNA virus belonging to the genus *Bromovirus.* The primary apple mosaic virus hosts are hops (*Humulus lupulus L.*) and apples (*Malus domestica Malus*), and hops infected with apple mosaic virus are known to have reduced cone yields, as well as lower contents of α acids that are the necessary bitter substances for beer brewing.

The "specimen" used for the detection method described above may be, for example, plant leaves, stems, roots or fruit, although leaves and fruit are preferred for easier sampling, and leaves are more preferred. The sampling period for the specimen may be, for example, during cultivation, harvesting or storage.

In the extraction step, apple mosaic virus RNA present in plant cells surrounded by cell walls is extracted, and for example, the specimen may be physically homogenized in distilled water or buffer with a nearly alkaline pH, for extraction of the apple mosaic virus RNA.

The pH of the buffer is preferably about, for example, pH 8.0-pH 9.5, and the buffer used may be, for example, Tris/hydrochloride buffer, Tris/acetate buffer, phosphate buffer, carbonate buffer, borate buffer or the like. Methods for extraction of RNA and methods for extraction of genomic DNA from plants are suited for more efficient extraction of the apple mosaic virus RNA from the specimen, and such methods are described in the genetic engineering protocols found in, for example, Molecular cloning (Maniatis et al., 1989, Cold Spring Harbor Laboratory Press). Extraction buffer containing guanidine thiocyanate is more preferred for RNA extraction methods while extraction buffer containing cetyl trimethylammonium bromide (CTAB) is more preferred for genomic DNA extraction methods.

The homogenization may be accomplished in any manner that crushes the cell walls of the specimen to allow extraction of the apple mosaic virus RNA present in the plant cells, and for example, it may be accomplished using a Polytron homogenizer, Teflon homogenizer, mortar or the like. If the specimen is homogenized in a frozen state, it will be possible to more efficiently crush the cell walls of the specimen and more easily extract the apple mosaic virus RNA present in the cells. Also, when RNA extraction buffer containing guanidine thiocyanate or genomic DNA extraction buffer containing CTAB as described above is used, the apple mosaic virus genomic RNA present in the cells can be extracted simply by repeated stirring with a vortex mixer or the like.

The apple mosaic virus genomic RNA extracted in the extraction step may be precipitated by alcohol precipitation and recovered and the buffer replaced with one suitable for RT-LAMP, but when the apple mosaic virus RNA has been extracted in distilled water or buffer with a nearly weak alkaline pH, it may be used directly for the subsequent amplification step.

In the amplification step, two different oligonucleotides consisting of the nucleotide sequences represented by SEQ ID NO: 2 and 4 of the Sequence Listing are used as primers for reverse transcription reaction using the apple mosaic virus RNA extracted in the extraction step as template, and then a primer set containing 4 different oligonucleotides consisting of the nucleotide sequences represented by SEQ ID NO: 1-4 of the Sequence Listing is used for repeated isothermal gene amplification reaction by Loop-mediated isothermal AMPlification (LAMP) using the cDNA obtained in the reverse transcription reaction as template.

The method for amplification of cDNA in the amplification step is known as RT-LAMP, and it can amplify a partial sequence of cDNA in the target region of apple mosaic virus genomic RNA by simultaneously accomplishing reverse transcription in which cDNA is synthesized by RNA, and isothermal gene amplification by LAMP, at constant temperature.

Fig. 1 is a diagram showing the positional relationship between the primer set comprising 4 different oligonucleotides consisting of the nucleotide sequences represented by SEQ ID NO: 1-4 of the Sequence Listing, and the target region of apple mosaic virus genomic RNA.

SEQ ID NO: 7 of the Sequence Listing represents a nucleotide sequence of cDNA in the target region of apple mosaic virus genomic RNA.

The oligonucleotide consisting of the nucleotide sequence represented by SEQ ID NO: 1 of the Sequence Listing (ApMV FIP primer) is an FIP primer, and it may be designed so that it carries the F2 region which is the sequence complementary to the F2c region at the 3'-end and the same sequence as the F1c region at the 5'-end.

The oligonucleotide consisting of the nucleotide sequence represented by SEQ ID NO: 2 of the Sequence Listing (ApMV-BIP primer) is a BIP primer, and it may be designed so that it carries the B2 region which is the sequence complementary to the B2c region at the 3'-end and the same sequence as the B1c region at the 5'-send.

The oligonucleotide consisting of the nucleotide sequence represented by SEQ ID NO: 3 of the Sequence Listing (ApMV-F3 primer) is an F3 primer, and it may be designed so that it carries the F3 region which is the sequence complementary to the F3c region.

The oligonucleotide consisting of the nucleotide sequence represented by SEQ ID NO: 4 of the Sequence Listing (ApMV-B3 primer) is a B3 primer, and it may be designed so that it carries the B3 region which is the sequence complementary to the B3c region.

In the cDNA amplification reaction by RT-LAMP, a reaction mixture containing the apple mosaic virus RNA extracted in the extraction step, a primer set containing 4 different oligonucleotides consisting of the nucleotide sequences represented by SEQ ID NO: 1-4 of the Sequence Listing, reverse transcriptase, strand displacing DNA synthase, dNTPs (dATP, dTTP, dGTP and dCTP) and buffer is allowed to stand for a prescribed time period at a constant temperature.

The temperature is preferably between 50°C and 75°C, more preferably between 60°C and 65°C, and even more preferably 65°C. A stationing time of at least 15 minutes will allow detection of cDNA amplification, but it is preferably between 15 minutes and 1 hour, and more preferably between 20 minutes and 40 minutes.

The reverse transcriptase, strand displacing DNA synthase, dNTPs and buffer may be reagents included in, for example, a Loopamp RNA Amplification Reagent Kit (product of Eiken Chemical Co., Ltd.).

In the amplification step, it is preferred to use a primer set with 6 different oligonucleotides consisting of the nucleotide sequences represented by SEQ ID NO: 1-6 of the Sequence Listing, which is obtained by further adding 2 different oligonucleotide primers consisting of the nucleotide sequences represented by SEQ ID NO: 5 and 6 of the Sequence Listing, instead of a primer set containing 4 different oligonucleotides consisting of the nucleotide sequences represented by SEQ ID NO: 1-4 of the Sequence Listing.

The oligonucleotide consisting of the nucleotide sequence represented by SEQ ID NO: 5 of the Sequence Listing (ApMV-LoopF primer) and the oligonucleotide consisting of the nucleotide sequence represented by SEQ ID NO: 6 of the Sequence Listing (ApMV-LoopB primer) are Loop primers having sequences complementary to the single-stranded portions of the 5'-end loops of the dumbbell structure serving as the origins in the amplification reaction. ApMV-LoopF may be designed so as to have the sequence complementary to the sequence between the F1 region and F2 region in Fig. 1, and ApMV-LoopB may be designed so as to have the sequence complementary to the sequence between the B1 region and B2 region in Fig. 1. By using ApMV-LoopF and ApMV-LoopB, it is possible to increase the number of origins for DNA synthesis, thereby increasing amplification efficiency and allowing the time required for amplification to be shortened to 1/3-1/2.

In the judging step, apple mosaic virus may be judged to be present if amplification of cDNA containing repeats of the nucleotide sequence represented by SEQ ID NO: 8 of the Sequence Listing occurs in the amplification step.

The nucleotide sequence represented by SEQ ID NO: 8 of the Sequence Listing is cDNA comprising a partial sequence of the target region of apple mosaic virus genomic RNA, and it is the core sequence that is repeatedly amplified in RT-LAMP using the aforementioned primer set. The nucleotide sequence of genomic RNA of apple mosaic virus complementary to this core sequence, and the nucleotide sequences of genomic RNA of apple mosaic virus to which the 4 different oligonucleotides consisting of the nucleotide sequences represented by SEQ ID NO: 1-4 of the Sequence Listing anneal, are partial sequences of the gene for the coat protein of apple mosaic virus, and the regions of the genomic RNA of apple mosaic virus containing these nucleotide sequences are suitable target regions for amplification of cDNA by RT-LAMP. Consequently, the presence of apple mosaic virus can be specifically detected by confirming whether or not amplified cDNA is present.

The presence of apple mosaic virus may be judged by, for example, unaided visual observation of the reaction mixture after amplification of the cDNA by RT-LAMP, determining apple mosaic virus to be present if opacity of the reaction mixture is confirmed. Alternatively, a fluorescent intercalator may be added to the reaction mixture and apple mosaic virus judged to be present if UV irradiation produces luminescence indicating amplification of the cDNA.

In order to judge the presence of apple mosaic virus more accurately, for example, the amplified cDNA may be digested with restriction enzyme AluI or TaqI having a site in the nucleotide sequence represented by SEQ ID NO: 8 of the Sequence Listing, and then electrophoresis performed, judging apple mosaic virus to be present if 134 bp and 75 bp DNA fragments or a 209 bp DNA fragment are confirmed.

The detection primer set for apple mosaic virus according to the invention is characterized by comprising 4 different oligonucleotides consisting of the nucleotide sequences represented by SEQ ID NO: 1-4 of the Sequence Listing or by comprising 6 different oligonucleotides consisting of the nucleotide sequences represented by SEQ ID NO: 1-6 of the Sequence Listing.

These primer sets can be synthesized with a DNA synthesizer based on the nucleotide sequence information of SEQ ID NO: 1-6 of the Sequence Listing.

The reagents necessary for the detection method for apple mosaic virus according to the invention may be pre-packaged as a kit. Specifically, a kit may be provided containing the primer set comprising 4 different oligonucleotides consisting of the nucleotide sequence represented by SEQ ID NO: 1-4 of the Sequence Listing or 6 different oligonucleotides consisting of the nucleotide sequence represented by SEQ ID NO: 1-6 of the Sequence Listing, the 4 different dNTPs (dATP, dCTP, dGTP and dTTP) that are substrates for nucleic acid synthesis, reverse transcriptase that synthesizes cDNA from RNA, strand displacing DNA synthase with strand displacing activity, buffer that creates the conditions suitable for the enzyme reaction, salts (magnesium salt or manganese salt, for example) as cofactors, protective agents to stabilize the enzyme or template, and reagents required for detection of the reaction product as necessary.

The kit may further contain a positive control to confirm normal progression of RT-LAMP reaction with the primers of the invention. The positive control may be, for example, DNA containing a region amplified by the primer of the invention.

### Examples

The present invention will now be explained in greater detail using examples, with the understanding that the invention is not limited to these examples.

### (Example 1) Detection of apple mosaic virus by RT-LAMP

### 1) Preparation of hop tissue samples for use in detection of apple mosaic virus

The specimen for detection of apple mosaic virus was obtained by removing the roots from hop test tube seedlings grown from suckers of hops infected with apple mosaic virus, while the control specimen was obtained by removing the roots from virus-free hop test tube seedlings, with hop tissue samples being prepared from each specimen.

The apple mosaic virus detection specimen and the control specimen were each frozen in liquid nitrogen and pulverized with a mortar until the tissue became powdery, and then a 5-fold amount of distilled water by weight was added to the specimen for suspension. The apple mosaic virus detection specimen and control specimen suspensions obtained in this manner were used as hop tissue samples for the following experiment.

### 2) Preparation of RNA samples

To each hop tissue sample (40 µL each) there was added 160 µL each of 2 × CTAB solution (2% cetyltrimethylammonium bromide, 20 mM EDTA, 1.4 M NaCl, 5% β-mercaptoethanol, 0.1 M Tris, pH 9.5), and the mixture was warmed at 65°C for 20 minutes. It was then centrifuged at 15,000 rpm for 10 minutes to produce a residue which was removed, and the supernatant was transferred to a new Eppendorf tube. Next, 200 µL of isopropanol was added and mixed therewith and the precipitate resulting from centrifugal separation at 15,000 rpm for 10 minutes was recovered as RNA, and after rinsing with 70% ethanol and drying, it was dissolved in 40 µL of sterilized water to obtain an RNA sample.

The RNA sample of the apple mosaic virus detection specimen and the RNA of the control specimen were each serially diluted 10³-, 10⁴-, 10⁵-, 10⁶- and 10⁷-fold using sterilized water, and 2 µL thereof was provided as RNA-diluted sample for cDNA amplification by RT-LAMP. The RNA-diluted samples corresponded to 1/(2 × 10⁴), 1/(2 × 10⁵), 1/(2 × 10⁶), 1/(2 × 10⁷) and 1/(2 × 10⁸) of the hop tissue samples.

### 3) Primers used for RT-LAMP

For the primers there were used ApMV-FIP primer (SEQ ID NO: 1) as the FIP primer, ApMV-BIP primer (SEQ ID NO: 2) as the BIP primer, ApMV-F3 primer (SEQ ID NO: 3) as the F3 primer, ApMV-B3 primer (SEQ ID NO: 4) as the B3 primer and ApMV-LoopF (SEQ ID NO: 5) and ApMV-LoopB (SEQ ID NO: 6) as the Loop primers. The 6 different primers were used for amplification of cDNA by RT-LAMP, thus allowing amplification of cDNA containing repeats of the nucleotide sequence represented by SEQ ID NO: 8 of the Sequence Listing, as a partial sequence of the cDNA of the apple mosaic virus coat protein gene.

### 4) Amplification of cDNA by RT-LAMP

Amplification of the cDNA by RT-LAMP was accomplished using a Loopamp RNA Amplification Reagent Kit (product of Eiken Chemical Co., Ltd.). Specifically, following the manufacturer's protocol, each RNA-diluted sample obtained by serial dilution (2 µl) was added to the reaction mixture, and then the 6 different primers, reverse transcriptase, strand displacing DNA synthase, dNTPs and buffer were added and the mixture was allowed to stand at 65°C for 50 minutes.

### 5) Detection of amplified cDNA

Since amplification of cDNA by RT-LAMP leads to reaction of free pyrophosphoric acid with magnesium ion to form magnesium pyrophosphate, the cDNA amplification can be determined based on the opacity of the reaction mixture. The cDNA amplified by the RT-LAMP cDNA amplification is the nucleotide sequence represented by SEQ ID NO: 8 of the Sequence Listing which corresponds to a partial sequence of the cDNA of the apple mosaic virus coat protein gene, and therefore opacity of the reaction mixture was judged as the presence of apple mosaic virus.

Table 1 shows the results of detecting apple mosaic virus by RT-LAMP using the RNA-diluted samples of the apple mosaic virus detection specimen and the RNA-diluted samples of the control specimen.

**[Table 1]**

| Degree of dilution from hop tissue sample | 2 × 10⁴ | 2 × 10⁵ | 2 × 10⁶ | 2 × 10⁷ | 2 × 10⁸ |
|---|---|---|---|---|---|
| Apple mosaic virus detection specimen | Opaque | Opaque | Opaque | ND | ND |
| Control specimen | ND | ND | ND | ND | ND |

| | | | | | |
|---|---|---|---|---|---|
| ND: No opacity observed. | | | | | |

No apple mosaic virus was detected in any of the RNA-diluted samples of the control specimen, but apple mosaic virus was detected in the RNA-diluted samples of the apple mosaic virus detection specimen even with a hop tissue sample at 2 × 10⁷-fold dilution. This demonstrated that when detection is carried out by RT-LAMP, apple mosaic virus is detected only in the specimens for apple mosaic virus detection which were infected with apple mosaic virus, and the detection sensitivity was found to be much higher than detection by ELISA, described hereunder.

Nevertheless, since opacity of the reaction mixture was observed even with nonspecific amplification of cDNA by RT-LAMP, a confirmation test was carried out by the following experiment. Specifically, 1 µL of each reaction mixture for which cDNA amplification was detected in the cDNA amplification by RT-LAMP using the RNA-diluted sample of the apple mosaic virus detection specimen, was digested with restriction enzyme AluI or TaqI and fractionated by polyacrylamide gel electrophoresis, and it was confirmed whether or not 134 bp and 75 bp DNA fragments or a 209 bp DNA fragment was detected. AluI and TaqI sites are present in the nucleotide sequence represented by SEQ ID NO: 8 of the Sequence Listing, and therefore digestion with restriction enzyme AluI or TaqI allows the cDNA amplified by RT-LAMP to be detected as 134 bp and 75 bp DNA fragments or a 209 bp DNA fragment. The electrophoresed gel may be dipped in ethidium bromide solution for 10 minutes to stain the fractionated DNA fragments, and then irradiated with ultraviolet rays for visualization of the DNA bands.

Fig. 2 is a gel diagram showing the band pattern obtained with restriction enzyme AluI or TaqI for digestion of cDNA amplified by RT-LAMP using an RNA-diluted sample of an apple mosaic virus detection specimen, followed by polyacrylamide gel electrophoresis.

As a result, opacity was observed in the cDNA amplification reaction by RT-LAMP, and 134 bp and 75 bp DNA fragment bands or a 209 bp DNA fragment band were detected by digestion with restriction enzyme AluI or TaqI, for all of the cDNA in each reaction mixture in which apple mosaic virus was detected in Table 1. Minor bands of 155 bp, 98 bp and 53 bp are also seen in the gel of the electrophoresed lanes of the cDNA digested with TaqI, in addition to the major band of 209 bp, but since linked cDNA partial sequences of the target region are amplified in cDNA amplification by LAMP, the presence of the minor bands of 155 bp, 98 bp and 53 bp in addition to the major band of 209 bp may be considered a proper result.

Thus, it was demonstrated that when opacity indicating amplification of cDNA by RT-LAMP was observed in Example 1, it was not nonspecific cDNA amplification but rather specific amplification of a portion of the apple mosaic virus coat protein gene, showing that the detection of apple mosaic virus was clearly dependent on the presence of apple mosaic virus.

This suggested that the detection method for apple mosaic virus by RT-LAMP described in Example 1 is a very highly sensitive method compared to the detection of apple mosaic virus by ELISA described hereunder, and that it allows the presence of apple mosaic virus to be easily judged merely by visually determining the opacity of the reaction mixture.

### (Comparative Example 1) Detection of apple mosaic virus by ELISA

### 1) Preparation of ELISA plate for detection of apple mosaic virus Anti-apple mosaic virus antibody (provided by Agria, US) that specifically recognizes apple mosaic virus was diluted to 1 µg/mL with 0.05 M SCB buffer (1.59 g/L Na₂CO₃, 2.93 µ/L NaHCO₃, pH 9.6) and then dispensed into a 96-well microplate at 0.2 mL each and stationed at 37°C for 4 hours. Next, 0.02 M PBS-T (8.0 g/L NaCl, 0.2 g/L KH₂PO₄, 2.9 g/L Na₂HPO₄ · 12H₂O, 0.2 g/L KCl, 0.5 mL/L Tween-20, pH 7.4) was used for rinsing 5 times at 5 minute intervals, and the obtained antibody solid phase plate was used as an ELISA plate for detection of apple mosaic virus, in the following test.

### 2) Detection by ELISA

To hop tissue samples (40 µL each) prepared from the apple mosaic virus detection specimens and control specimens mentioned in Example 1 there was added 160 µL of distilled water, and then 200 µL of PBS-T/PVP solution (16.0 g/L NaCl, 0.4 g/L KH₂PO₄, 5.8 g/L Na₂HPO₄ · 12H₂O, 0.4 g/L KC1, 1.0 mL/L Tween-20, 40 g/L polyvinylpyrrolidone, pH 7.4) was further added and mixed therewith, the mixture was centrifuged at 15,000 rpm for 10 minutes and the supernatant was recovered for use as a protein sample (200 µL).

The apple mosaic virus detection specimen and control specimen protein samples were each serially diluted 10-, 10² - and 10³-fold with PBS-T/PVP solution and 200 µL of each was provided as a protein-diluted sample for ELISA. The non-diluted protein sample corresponded to 1/2 of the hop tissue sample and the protein-diluted samples serially diluted 10-, 10²- and 10³-fold corresponded sample.

The protein sample and each of the protein-diluted samples (200 µL each) were added to the aforementioned ELISA plate and stationed overnight. After using 0.02 M PBS-T for rinsing 5 times at 5 minute intervals, 200 µL of alkaline phosphatase-labeled anti-apple mosaic virus antibody (Agria, US) diluted 1000-fold with 0.02 M PBS-T was added to each and the mixtures were further stationed at 37°C for 3 hours. Next, each well of the ELISA plate was rinsed 5 times at 5 minute intervals using 0.02 M PBS-T, and then 200 µL of substrate solution (10% diethanolamine, 1 g/L disodium p-nitrophenylphosphate) was added and reaction was conducted for a fixed time at room temperature, after which the absorbance at 405 nm was measured.

Table 2 shows the results of detecting apple mosaic virus by ELISA using the protein sample and each protein-diluted sample of the apple mosaic virus detection specimen, and the protein sample and each protein-diluted sample of the control specimen.

**[Table 2]**

| Degree of dilution from hop tissue sample | 2 | 20 | 2 × 10² | 2 × 10³ |
|---|---|---|---|---|
| Apple mosaic virus detection specimen | Opaque | Opaque | ND | ND |
| Control specimen | ND | ND | ND | ND |

| | | | | |
|---|---|---|---|---|
| ND: No opacity observed. | | | | |

No apple mosaic virus was detected in any of the protein sample and each protein-diluted sample of the control specimen, but apple mosaic virus was detectable up to 20-fold dilution of the hop tissue sample, in the protein sample and each protein-diluted sample of the apple mosaic virus detection specimen. This indicated that, in ELISA, apple mosaic virus is detected only in apple mosaic virus detection specimens infected with apple mosaic virus and that apple mosaic virus can be properly detected, but that the detection sensitivity is at least 10⁶-fold (1,000,000-fold) lower compared to detection by RT-LAMP as described above.

### Industrial Applicability

According to the invention, it is possible to detect apple mosaic virus specifically and with high sensitivity in any season including during cultivation, harvesting and storage, regardless of the number of specimens or the location of evaluation. Moreover, the detection primer set and detection kit for apple mosaic virus according to the invention may be suitably used for RT-LAMP, thus allowing simple and high-sensitivity detection of apple mosaic virus.

## Claims

1. A detection method for apple mosaic virus that comprises:
(a) an extraction step in which apple mosaic virus RNA is extracted from a specimen,
(b) an amplification step in which a primer set containing 4 different oligonucleotides consisting of the nucleotide sequences represented by SEQ ID NO: 1-4 of the Sequence Listing is used for amplification of cDNA by Reverse Transcription-Loop-mediated isothermal AMPlification (RT-LAMP) using the RNA as template, and
(c) a judging step in which apple mosaic virus is judged to be present when amplification of cDNA containing the nucleotide sequence represented by SEQ ID NO: 8 of the Sequence Listing has occurred in the amplification step.

2. A detection method according to claim 1, wherein the amplification step is a step in which a primer set containing 6 different oligonucleotides consisting of the nucleotide sequences represented by SEQ ID NO: 1-6 of the Sequence Listing is used for amplification of cDNA by Reverse Transcription-Loop-mediated isothermal AMPlification (RT-LAMP) using the RNA as template.

3. A detection primer set for apple mosaic virus that comprises 4 different oligonucleotides consisting of the nucleotide sequences represented by SEQ ID NO: 1-4 of the Sequence Listing.

4. The detection primer set according to claim 3, wherein the detection primer set for apple mosaic virus comprises 6 different oligonucleotides consisting of the nucleotide sequences represented by SEQ ID NO: 1-6 of the Sequence Listing.

5. A detection kit for apple mosaic virus which contains a primer set according to claim 3 or 4, reverse transcriptase, a strand displacing DNA synthase, dNTPs and a buffer.

## Patentansprüche

1. Nachweisverfahren für das Apfelmosaikvirus, das umfasst:
(a) einen Extraktionsschritt, in dem die Apfelmosaikvirus-RNA aus einer Probe extrahiert wird;
(b) einen Amplifikationsschritt, in dem ein Primerset, das 4 verschiedene Oligonucleotide beinhaltet, bestehend aus den Nucleotidsequenzen wie in SEQ ID NO: 1-4 des Sequenzprotokolls gezeigt, zur Amplifikation der cDNA durch Reverse Transcription-Loop-mediated isothermal AMPlification (RT-LAMP) unter Verwendung der RNA als Matrize verwendet wird; und
(c) einen Auswertschritt, in dem das Apfelmosaikvirus als vorhanden bewertet wird, wenn die Amplifikation der cDNA, enthaltend die Nucleotidsequenz wie durch SEQ ID NO: 8 des Sequenzprotokolls gezeigt, im Amplifikationsschritt vorgekommen ist.

2. Nachweisverfahren gemäß Anspruch 1, wobei der Amplifikationsschritt ein Schritt ist, in dem ein Primerset, das 6 verschiedene Oligonucleotide beinhaltet, bestehend aus den Nucleotidsequenzen wie in SEQ ID NO: 1-6 des Sequenzprotokolls gezeigt, zur Amplifikation der cDNA durch Reverse Transcription-Loop-mediated isothermal AMPlification (RT-LAMP) unter Verwendung der RNA als Matrize verwendet wird.

3. Nachweisprimerset für das Apfelmosaikvirus, umfassend 4 verschiedene Oligonucleotide, die aus den Nucleotidsequenzen wie in SEQ ID NO: 1-4 des Sequenzprotokolls gezeigt bestehen.

4. Nachweisprimerset gemäß Anspruch 3, wobei das Nachweisprimerset für das Apfelmosaikvirus 6 verschiedene Oligonucleotide umfasst, die aus den Nucleotidsequenzen wie durch SEQ ID NO: 1-6 des Sequenzprotokolls gezeigt bestehen.

5. Nachweiskit für das Apfelmosaikvirus, das einen Primerset gemäß Anspruch 3 oder 4, reverse Transkriptase, eine Strang-verdrängende DNA-Synthase, dNTPs und einen Puffer beinhaltet.

## Revendications

1. Méthode de détection du virus mosaïque de la pomme comprenant :
(a) une étape d'extraction dans laquelle l'ARN du virus mosaïque de la pomme est extrait d'un spécimen,
(b) une étape d'amplification dans laquelle un jeu d'amorces, contenant 4 oligonucléotides différents consistant en les séquences de nucléotides représentées par SEQ ID NO: 1-4 de la liste de séquences, est utilisé pour l'amplification d'ADNc par AMPlification isotherme médiée par transcription inverse en boucle (RT-LAMP) en utilisant l'ARN comme matrice, et
(c) une étape de jugement dans laquelle le virus mosaïque de la pomme est jugé comme étant présent quand l'amplification d'ADNc contenant la séquence de nucléotides représentée par SEQ ID NO: 8 de la liste de séquences a eu lieu pendant l'étape d'amplification.

2. Méthode de détection selon la revendication 1, dans laquelle l'étape d'amplification est une étape dans laquelle un jeu d'amorces contenant 6 oligonucléotides différents, consistant en les séquences de nucléotides représentées par SEQ ID NO: 1-6 de la liste de séquences, est utilisé pour l'amplification d'ADNc par AMPlification isotherme médiée par transcription inverse en boucle (RT-LAMP) en utilisant l'ARN comme matrice.

3. Jeu d'amorces de détection pour le virus mosaïque de la pomme qui comprend 4 oligonucléotides différents, consistant en les séquences de nucléotides représentées par SED ID NO: 1-4 de la liste de séquences.

4. Jeu d'amorces de détection selon la revendication 3, dans lequel le jeu d'amorces de détection pour le virus mosaïque de la pomme comprend 6 oligonucléotides différents, consistant en les séquences de nucléotides représentées par SEQ ID NO: 1-6 de la liste de séquences.

5. Kit de détection pour le virus mosaïque de la pomme qui contient un jeu d'amorces selon la revendication 3 ou 4, de la transcriptase inverse, de la synthase d'ADN capable de déplacer des brins, des dNTPs et un tampon.


### SEQUENCE LISTING

<110> Sapporo Breweries Ltd.
<120> Detection method of Apple mosaic virus, primer set and kit for detection of Apple mosaic virus
<130> R1953 EP S3
<140> EP 07 83 2502.4 <141> 2007-11-26
<150> JP 2006-324371 <151> 2006-11-30
<160> 8
<170> PatentIn version 3.1
<210> 1
   <211> 40
   <212> DNA
   <213> Artificial sequence
<220>
   <221> source
   <223> /note= "Description of artificial sequence: primer"
<400> 1
   taacctccca agctgtcctc ctcgctcaag cgaacccgaa 40
<210> 2
   <211> 40
   <212> DNA
   <213> Artificial sequence
<220>
   <221> source
   <223> /note= "Description of artificial sequence: primer"
<400> 2
   gcccgaatgt ggaaccgaag ataacttgcc tgcgaccgta 40
<210> 3
   <211> 20
   <212> DNA
   <213> Artificial sequence
<220>
   <221> source
   <223> /note= "Description of artificial sequence: primer"
<400> 3
   acgaatagac ctgctcctcc 20
<210> 4
   <211> 20
   <212> DNA
   <213> Artificial sequence
<220>
   <221> source
   <223> /note= "Description of artificial sequence: primer"
<400> 4
   cggaagacat cggcaaagtc 20
<210> 5
   <211> 21
   <212> DNA
   <213> Artificial sequence
<220>
   <221> source
   <223> /note= "Description of artificial sequence: primer"
<400> 5
   tcccttcacg ggattcttac c 21
<210> 6
   <211> 20
   <212> DNA
   <213> Artificial sequence
<220>
   <221> source
   <223> /note= "Description of artificial sequence: primer"
<400> 6
   cacagggctt tgagcagtcg 20
<210> 7
   <211> 230
   <212> DNA
   <213> Apple mosaic virus
<400> 7
   acgaatagac ctgctcctcc gaagcgcgcc gttattcgcg ctcaagcgaa cccgaacaag 60
   ggtaagaatc ccgtgaaggg aggtccgtcc gtgaggagga cagcttggga ggttagaggc 120
   ccgaatgtgg aaccgaagat tccaaagggt cacagggctt tgagcagtcg agaagtgact 180
   gctacggtcg caggcaagtt tgtgaatatt gactttgccg atgtcttccg 230
<210> 8
   <211> 185
   <212> DNA
   <213> Apple mosaic virus
<400> 8
   aatgctgggc tagaaacagg ccggccagcg ctcaagccca cacctgacat gctgcctgat 60
   cctaccaacc catataacaa accctcatta gatgctttgt tgatgataaa gccaagagtt    120
   gtgtctaaca atatggctac ctctgaggat atgatgaaga tctgcgttga tcttgagggg    180
   ttggg                                                                185